# EUROPEAN PATENT APPLICATION

(11) **EP 3 015 859 A1**
(43) Date of publication of application: **04.05.2016**
(21) Application number: 14816767.9
(22) Date of filing: 23.05.2014
(51) Int. Cl.: G01N 33/48, G01N 33/49, G01N 33/72

(54) **SPECIMEN CONTAINING UNIT, SPECIMEN MEASUREMENT CASSETTE, SPECIMEN MEASUREMENT UNIT, AND SPECIMEN MEASUREMENT DEVICE**

(30) Priority: 25.06.2013 KR 20130073032; 25.06.2013 KR 20130073031
(71) Applicant: Infopia Co., Ltd., Anyang-si, Gyeonggi-do 431-836 (KR)
(72) Inventor: BAE, Byeong Woo, Anyang-si Gyeonggi-do 431-774 (KR); CHON, Chan Hee, Anyang-si Gyeonggi-do 431-719 (KR); KIM, Hyo Jeong, Anyang-si Gyeonggi-do 431-735 (KR)
(74) Representative: Gill, Siân Victoria
(86) International application number: PCT/KR2014/004620
(87) International publication number: WO 2014/208894

(57) **Abstract**

A specimen containing unit according to one embodiment of the present invention relates to a structure for dipping a specimen into a reagent so as to react with the reagent, and the specimen containment unit comprises: a body portion rotatable by external force; an extension portion formed to be extended from one surface of the body portion at a predetermined length along a longitudinal direction; and a specimen containment portion provided to an end portion of the extension portion and including a containment hole formed to be penetrated while maintaining at a predetermined angle in the longitudinal direction so as to contain a specimen by a capillary phenomenon.

## Description

### [Technical Field]

The present invention relates to a specimen-containing unit, a specimen measurement cassette, a specimen measurement unit, and a specimen measurement device, and more particularly, to a specimen-containing unit, a specimen measurement unit, and a specimen measurement device, all of which are able to achieve accurate measurement results by assaying results obtained by reaction of a reagent with a specimen.

### [Background Art]

In recent years, measuring concentrations of compounds used to analyze anesthetics or harmful chemicals has been efficiently used for medical diagnoses or treatments using drugs in a medical or environmental field. Among these, interest in measuring concentrations of biological samples used in fields of medical diagnoses and treatments has continuously grown with an increase in humans' desire to become free from various diseases. In particular, there is an increasing interest in a glycated hemoglobin test that may be used to measure blood sugar associated with diabetes since a sample is measured once to check an average of blood sugar levels maintained for a relatively long period of time.

Hemoglobin Alc (HbA1c) is referred to as a glycated hemoglobin, which is present in human red blood cells as one type of hemoglobin. When blood sugar (glucose) in blood increases, some of the glucose in blood binds to hemoglobin. The hemoglobin bound to such glucose is referred to as glycated hemoglobin. An average of blood sugar levels maintained for the past 2 to 3 months may be deduced by means of such a glycated hemoglobin test. In this case, the glycated hemoglobin test has an advantage in that it may be used to draw and test blood regardless of meal times.

Meanwhile, US Registered Patent No. 6,300,142 discloses an apparatus for assaying analytes by allowing a test sample to react with a first reagent through a first inlet port and sequentially to react with a second reactant through a second inlet port so as to assay the analytes present in the sample. In this case, the measurements should be sequentially carried out over time.

Also, a researcher performing such measurements should intervene in a measurement process by sequentially injecting test samples. In addition, such an apparatus has a drawback in that the measurements are complicated since beads bound to glycated hemoglobin should be filtered out, which results in an increase in measurement time.

That is, researchers may be inconvenienced since such an experiment requires direct intervention by the researchers in several processes. Also, the intervention by the researchers may result in a complicated measurement process and a delay in measurement time.

Also, Korean Registered Patent No. 0798471 discloses a cassette which includes a first accommodation area configured to accommodate a first reagent, a second accommodation area configured to accommodate a second reagent, a reaction area configured to allow a blood sample to react with the first or second reagent, and a measurement area configured to measure the total amount of hemoglobin or glycated hemoglobin in the blood sample, characterized in that the reaction area and the measurement area are separately formed according to a rotation angle position of the cassette.

However, the cassette has a drawback in that some of the first and second reagents may be mixed while the two reagents are input into the first and second accommodation areas, respectively. Also, since the cassette is manufactured by attaching an upper plate to a structural frame having an inner structure formed therein, the reagent may be leaked through a fine gap formed at an attachment site between the upper plate and the structural frame due to a capillary phenomenon, resulting in an error in measurement results.

Further, a certain amount of the blood sample is not collected when the blood sample is injected into the cassette from a cartridge including a blood collection portion configured to contain a blood sample. Also, an error in measurement results may occur when the amount of the sample exceeds a measurement limit. Therefore, there is a need for research on apparatuses capable of realizing more accurate measurement results.

### [Disclosure]

### [Technical Problem]

Therefore, it is an aspect of the present invention to provide a specimen-containing unit, a specimen measurement cassette, a specimen measurement unit, and a specimen measurement device, all of which are able to effectively implement a reaction between a specimen and a reagent for a short period of time and simultaneously to automatically accurately assay results obtained by the reaction of the reagent with the specimen while preventing mixing between the reagents and minimizing intervention by a researcher.

### [Technical Solution]

According to an aspect of the present invention, there is provided a specimen-containing unit having a structure in which a specimen is dipped in a reagent for a reaction with the reagent, which includes a body portion rotatable by an external force, an extension portion formed to extend from one surface of the body portion to a predetermined length in a longitudinal direction, and a specimen containment portion provided at an end portion of the extension portion and having a containment hole formed therethrough while being maintained at a predetermined angle in the longitudinal direction to contain the specimen due to a capillary phenomenon.

In the specimen-containing unit according to one exemplary embodiment of the present invention, the extension portion may be arranged outside the body portion based on the center of one surface thereof so that the reagent and the specimen contained in the containment hole are stirred as the specimen containment portion rotates with rotation of the body portion.

In the specimen-containing unit according to one exemplary embodiment of the present invention, a lower end portion of the specimen containment portion may be sharply formed through a lidding foil portion of an accommodation portion containing the reagent so that the specimen is dipped in the reagent.

In the specimen-containing unit according to one exemplary embodiment of the present invention, a direction of the containment hole may correspond to a tangential direction of an imaginary circle formed by an end portion of the extension portion as the extension portion rotates with rotation of the body portion.

The specimen-containing unit according to one exemplary embodiment of the present invention may be formed so that widths of the extension portion and the specimen containment portion correspond to each other.

In the specimen-containing unit according to one exemplary embodiment of the present invention, the specimen containment portion may be formed integrally with the extension portion.

According to another aspect of the present invention, there is provided a specimen measurement unit, which includes a specimen-containing unit, and a specimen measurement cassette including a first accommodation portion configured to store a first reagent in which the specimen containment portion is dipped to react with the specimen, a second accommodation portion partitioned from the first accommodation portion and configured to store a second reagent, and a measurement portion configured to assay results obtained by reaction of the specimen with the first reagent.

In the specimen measurement unit according to another exemplary embodiment of the present invention, the measurement portion may be configured to accommodate the second reagent after a predetermined time has elapsed after the first accommodation portion accommodates the specimen reacting with the first reagent.

In the specimen measurement unit according to another exemplary embodiment of the present invention, the inflow of the first reagent and the second reagent into the measurement portion may be implemented by self-loads of the first reagent and the second reagent.

In the specimen measurement unit according to another exemplary embodiment of the present invention, the first reagent and the second reagent may be prevented from flowing outwards by a first foil tap and a second foil tap configured to close bottom surfaces of the first accommodation portion and the second accommodation portion, respectively, and the first foil tap and the second foil tap are shifted from the bottom surfaces of the first accommodation portion and the second accommodation portion, respectively, by a specimen measurement device having the specimen measurement cassette installed therein to assay results obtained by reaction of the specimen with the first reagent and the second reagent, so that the first reagent and the second reagent flow into the measurement portion.

In the specimen measurement unit according to another exemplary embodiment of the present invention, each of the first foil tap and the second foil tap may be arranged so that one end of each of the first foil tap and the second foil tap is bent toward the other end.

The specimen measurement unit according to another exemplary embodiment of the present invention may further include an absorption portion disposed under the measurement portion to absorb a mixture of the specimen in which the results obtained by reaction of the specimen with the first reagent are completely assayed.

In the specimen measurement unit according to another exemplary embodiment of the present invention, the reaction of the specimen with the second reagent may be implemented at the measurement portion.

According to still another aspect of the present invention, there is provided a specimen measurement device, which includes a specimen measurement unit, and a specimen measurement body having the specimen measurement cassette installed therein, configured to assay results obtained by reaction of the specimen with the first reagent and the second reagent, and including an external force application portion configured to apply an external force to shift the first foil tap and the second foil tap from bottom surfaces of the first accommodation portion and the second accommodation portion, respectively.

In the specimen measurement device according to still another exemplary embodiment of the present invention, the external force application portion may apply the external force to one side end of the first foil tap and one side end of the second foil tap, respectively, in opposite directions.

In the specimen measurement device according to yet another exemplary embodiment of the present invention, the external force application portion may apply a pushing external force to one side end of the first foil tap and may apply a pulling external force to one side end of the second foil tap.

In the specimen measurement device according to yet another exemplary embodiment of the present invention, each of the first foil tap and the second foil tap may be arranged so that one end of each of the first foil tap and the second foil tap is bent toward the other end, and the external force application portion may apply a pulling or pushing external force to the other side ends of the first foil tap and the second foil tap.

In the specimen measurement device according to yet another exemplary embodiment of the present invention, the specimen measurement body may include a rotary force application portion configured to apply a rotary force to rotate the body portion after the specimen containment portion is dipped in the first reagent

### [Advantageous Effects]

The specimen-containing unit, the specimen measurement cassette, the specimen measurement unit, and the specimen measurement device according to the exemplary embodiments of the present invention can be useful in effectively realizing a reaction between a specimen and a reagent within a short period of time.

Also, the specimen-containing unit, the specimen measurement cassette, the specimen measurement unit, and the specimen measurement device according to the exemplary embodiments of the present invention can be useful in providing convenience to researchers since a place configured to store a reagent and a place in which the reagent reacts with a specimen are formed in one structure.

In addition, the specimen-containing unit, the specimen measurement cassette, the specimen measurement unit, and the specimen measurement device according to the exemplary embodiments of the present invention can be useful in preventing mixing between reagents to accurately assay results obtained by reaction of the specimen with the reagent.

Further, the specimen-containing unit, the specimen measurement cassette, the specimen measurement unit, and the specimen measurement device according to the exemplary embodiments of the present invention can be useful in shifting foil taps configured to introduce a reagent into a measurement portion using a simple method.

### [Description of Drawings]

FIG. 1 is a schematic cross-sectional view showing a specimen measurement device according to one exemplary embodiment of the present invention.
FIGS. 2 to 5 are schematic cross-sectional views for describing a process of assaying results obtained by reaction of a specimen using the specimen measurement device according to one exemplary embodiment of the present invention.
FIG. 6 is a schematic perspective view showing a specimen-containing unit according to one exemplary embodiment of the present invention.
FIG. 7 is a rear view for describing a direction of a specimen containment portion provided in the specimen-containing unit according to one exemplary embodiment of the present invention.
FIG. 8 is a schematic perspective view showing a modified specimen-containing unit according to one exemplary embodiment of the present invention.
FIG. 9 is a schematic cutaway perspective view showing a specimen measurement unit according to one exemplary embodiment of the present invention.
FIG. 10 is a schematic cutaway perspective view showing a modified specimen measurement unit according to one exemplary embodiment of the present invention.
FIG. 11 is a schematic cross-sectional view showing another modified specimen measurement unit according to one exemplary embodiment of the present invention.

### [Best Mode]

The specimen-containing unit according to one exemplary embodiment of the present invention has a structure in which a specimen is dipped in a reagent to react with the reagent, and includes a body portion rotatable by an external force, an extension portion formed to extend from one surface of the body portion to a predetermined length in a longitudinal direction, and a specimen containment portion provided at an end portion of the extension portion and having a containment hole formed therethrough while being maintained at a predetermined angle in the longitudinal direction to contain the specimen due to a capillary phenomenon.

### [Mode for Invention]

Hereinafter, preferred embodiments of the present invention will be described in detail referring to the accompanying drawings. However, it should be understood that the terms used in the specification and appended claims should not be construed as limited to general and dictionary meanings, but interpreted based on the meanings and concepts corresponding to technical aspects of the present invention on the basis of the principle that the inventor is allowed to define terms appropriately for the best description. Therefore, the description proposed herein is just a preferable example for the purpose of illustrations only, not intended to limit the scope of the invention, so it should be understood that other equivalents and modifications could be made thereto without departing from the scope of the invention.

Reference will now be made in detail to the embodiments of the present invention, examples of which are illustrated in the accompanying drawings, wherein like reference numerals refer to like elements throughout.

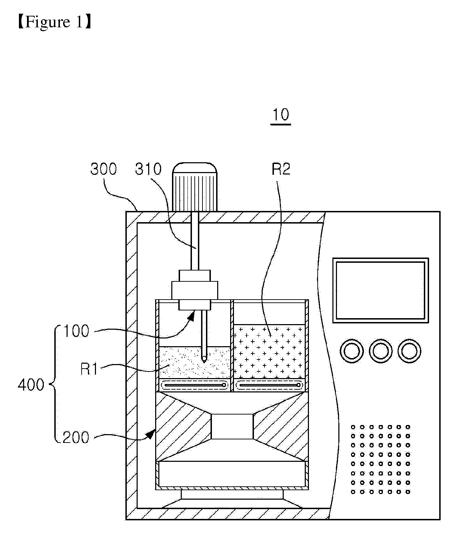

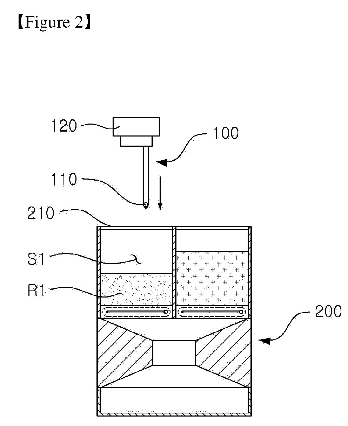

FIG. 1 is a schematic cross-sectional view showing a specimen measurement device according to one exemplary embodiment of the present invention, and FIGS. 2 to 5 are schematic cross-sectional views for describing a process of assaying results obtained by reaction of a specimen using the specimen measurement device according to one exemplary embodiment of the present invention.

Referring to FIG. 1, first of all, a specimen measurement device 10 according to one exemplary embodiment of the present invention may include a specimen-containing unit 100 for containing a specimen, a specimen measurement cassette 200 configured to store a first reagent R1 and a second reagent R2 for reacting with the specimen contained in the specimen-containing unit 100, and a specimen measurement body 300 having the specimen measurement cassette 200 installed therein to assay results obtained by reaction of the specimen with the first reagent R1 and the second reagent R2.

Here, the specimen-containing unit 100 and the specimen measurement cassette 200 may constitute a specimen measurement unit 400, and the specimen measurement device 10 may be intended to include the specimen measurement cassette 200 and the specimen measurement body 300 without using the specimen-containing unit 100.

The specimen measurement device 10 is a device capable of sequentially assaying results obtained by reaction of the specimen with the first reagent R1, and results obtained by reaction of the specimen with the second reagent R2. One example of the specimen measurement device 10 is a device for measuring glycated hemoglobin. Hereinafter, the measurement of glycated hemoglobin will be described by way of example.

As a device for measuring glycated hemoglobin, the specimen measurement device 10 may measure an amount of glycated hemoglobin by means of a measuring method using light reflection characteristics. When an amount of glycated hemoglobin in a blood sample as a specimen has to be measured, the measuring method may use characteristics of hemoglobin which specifically absorbs optical signals having a certain frequency range. Hereinafter, the measuring method will be described, as follows.

Referring to FIG. 2, first, a specimen-containing unit 100 in which a containment hole 110 can contain a specimen due to a capillary phenomenon is prepared, and the specimen is introduced into the containment hole 110.

The specimen contained in the containment hole 110 of the specimen-containing unit 100 may pass through a lidding foil portion 210 of the specimen measurement cassette 200 configured to store a first reagent R1, and may be inserted into a first accommodation portion S1 to react with the first reagent R1.

In this case, the specimen-containing unit 100 containing the specimen may rotate to enhance a speed of reaction between the first reagent R1 and the specimen. Here, a drive force for rotation may be provided by the specimen measurement body 300 having the specimen measurement cassette 200 installed therein.

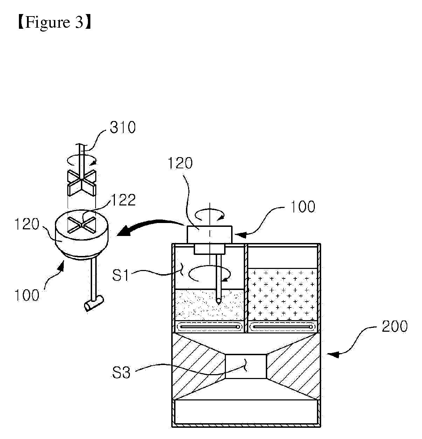

Referring to FIG. 3, when the specimen measurement cassette 200 is installed in the specimen measurement body 300, the specimen-containing unit 100 may rotate inside the first accommodation portion S1 by means of a rotary force application portion 310 provided inside the specimen measurement body 300.

The rotary force application portion 310 may be implemented to automatically move up and down. When the specimen-containing unit 100 is intended to rotate, the rotary force application portion 310 may be inserted into a groove 122 formed in a body portion 120 of the specimen-containing unit 100 while moving down.

That is, the rotation of the specimen-containing unit 100 by the rotary force application portion 310 may be carried out based on the principle of a flat-head or crosshead screwdriver.

However, the rotation of the specimen-containing unit 100 by the rotary force application portion 310 is not limited to being implemented by the principle of the screwdriver, and a method of rotating the specimen-containing unit 100 may be widely modified according to the intention of those skilled in the art.

When the reaction of the specimen with the first reagent R1 is completed with the rotation of the specimen-containing unit 100 after a predetermined time has elapsed, a reaction product of the first reagent R1 and the specimen may be allowed to flow into a measurement portion S3.

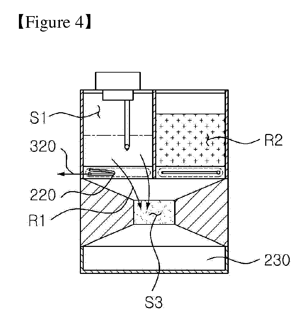

Referring to FIG. 4, the reaction product of the first reagent R1 and the specimen may flow into the measurement portion S3 by shifting a first foil tap 220 configured to close a bottom surface of the first accommodation portion S1, and the shifting of the first foil tap 220 may be implemented by an external force application portion 320 (indicated by arrow) provided at the specimen measurement body 300.

Here, the external force application portion 320 may push one side end of the first foil tap 220 so that the one side end of the first foil tap 220 is allowed to move toward the other side end thereof. As a result, a bottom surface of the first accommodation portion S1 is opened.

When the bottom surface of the first accommodation portion S1 is opened, the reaction product of the first reagent R1 and the specimen flows into the measurement portion S3, and the reaction results are assayed by a measurement sensor (not shown) provided at the specimen measurement body 300 using light reflection characteristics.

Here, the measurement sensor outputs an emission control signal to a light emitting element and converts an optical signal input from a light receiving element into an electric signal so as to assay the reaction results.

When the measurement is completed, a mixture of the specimen is absorbed by an absorption portion 230 disposed under the measurement portion S3, and the second reagent R2 is then allowed to flow into the measurement portion S3.

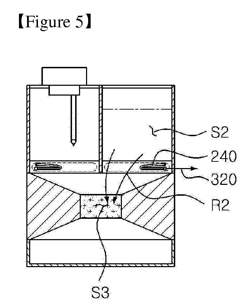

Referring to FIG. 5, the inflow of the second reagent R2 into the measurement portion S3 may be implemented by shifting a second foil tap 240 configured to close a bottom surface of a second accommodation portion S2. Here, the shifting of the second foil tap 240 may be implemented by the external force application portion 320.

The external force application portion 320 may pull one side end of the second foil tap 240 so that the one side end of the second foil tap 240 is allowed to move toward the other side end thereof. As a result, a bottom surface of the second accommodation portion S2 is opened.

The desired reaction results may be assayed by washing a mixture of the specimen with the second reagent R2 flowing in the measurement portion S3.

Hereinafter, a specimen measurement unit 400 including the above-mentioned specimen-containing unit 100 and specimen measurement cassette 200 will be described in further detail.

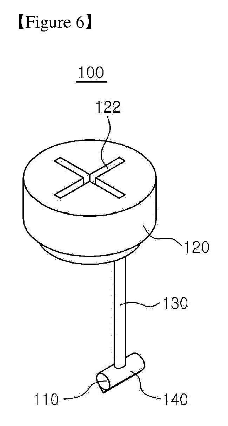

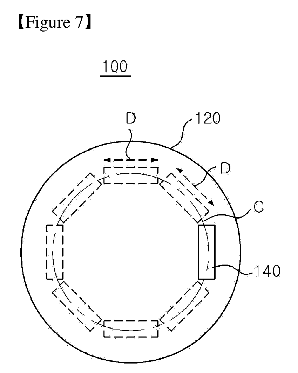

FIG. 6 is a schematic perspective view showing a specimen-containing unit according to one exemplary embodiment of the present invention, and FIG. 7 is a rear view for describing a direction of a specimen containment portion provided in the specimen-containing unit according to one exemplary embodiment of the present invention.

Referring to FIGS. 6 and 7, the specimen-containing unit 100 may, for example, be a type of a structure capable of containing a specimen as a blood sample. Here, the specimen may be dipped in a reagent to react with the reagent.

Here, the reagent may be the first reagent R1 stored in the first accommodation portion S1 of specimen measurement cassette 200, as described above with reference to FIGS. 1 to 5.

Specifically, the specimen-containing unit 100 may include a body portion 120 rotatable by an external force, an extension portion 130 formed to extend from one surface of the body portion 120 to a predetermined length in a longitudinal direction, and a specimen containment portion 140 provided at an end portion of the extension portion 130 and having a containment hole 110 formed therethrough to contain the specimen due to a capillary phenomenon.

The body portion 120 may be formed in an approximately cylindrical shape, and a groove 122 may be formed in the other surface of the body portion 120 so that the rotary force application portion 310 of the specimen measurement body 300 is inserted into the groove 122. In this case, the rotation of the body portion 120 by the rotary force application portion 310 may be carried out based on the principle of a screwdriver.

The extension portion 130 is a rotatable component which is interconnected with rotation of the body portion 120 by the rotary force application portion 310. In this case, the extension portion 130 may be arranged outside the body portion 120 based on the center of one surface thereof.

This is done to stir the specimen and the first reagent R1 contained in the containment hole 110 as the specimen containment portion 140 rotates with rotation of the body portion 120.

That is, the specimen containment portion 140 containing the specimen is dipped in the first reagent R1 stored in the first accommodation portion S1 of the specimen measurement cassette 200, and rotates in conjunction with the rotation of the body portion 120 so as to perform a reaction of the specimen with the first reagent R1.
In this case, this is done to minimize a reaction time through the rotation.

Here, the specimen containment portion 140 may maintain a direction of the containment hole 110 at a predetermined angle in a longitudinal direction so as to maximize efficiency of the reaction while the specimen containment portion 140 rotates in conjunction with the rotation of the body portion 120 in a state in which the specimen containment portion 140 is dipped in the first reagent R1.

Specifically, the direction of the containment hole 110 may be a direction corresponding to a tangential direction D of an imaginary circle C formed by an end portion of the extension portion 130 as the extension portion 130 rotates with rotation of the body portion 120. Therefore, the specimen contained in the containment hole 110 may be efficiently introduced into the first reagent R1.

Consequently, the blood sample contained in the containment hole 110 of the specimen containment portion 140 may be effectively hemolyzed by the first reagent R1 to flow out of the containment hole 110 due to the direction of the containment hole 110 and a position in which the extension portion 130 is formed.

Meanwhile, a lower end portion of the specimen containment portion 140 may be sharply formed through a lidding foil portion 210 configured to cover a top surface of the first accommodation portion S1 of the specimen measurement cassette 200 so that the specimen is dipped in the first reagent R1.Here, the first accommodation portion S1 contains the first reagent R1.

Therefore, the specimen-containing unit 100 according to one exemplary embodiment of the present invention may stably pass through the first accommodation portion S1 configured to store the first reagent R1 in a state in which the specimen is contained in the containment hole 110.

However, the entire lower end portion of the specimen containment portion 140 need not be sharply formed, but at least a portion of the lower end portion of the specimen containment portion 140 may be sharply formed.

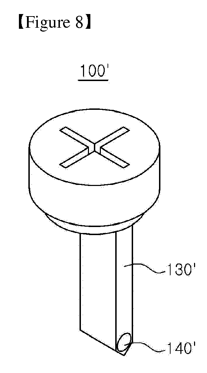

FIG. 8 is a schematic perspective view showing a modified specimen-containing unit according to one exemplary embodiment of the present invention.

Referring to FIG. 8, a specimen-containing unit 100' may be formed so that an extension portion 130' and a specimen containment portion 140' have widths corresponding to each other. For example, the extension portion 130' and the specimen containment portion 140' may be formed integrally with each other.

Consequently, the extension portion 130' and the specimen containment portion 140' may be physically the same components, and a lower end portion of the specimen containment portion 140' may be sharply formed.

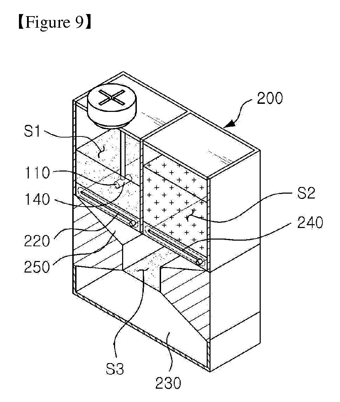

FIG. 9 is a schematic cutaway perspective view showing a specimen measurement unit according to one exemplary embodiment of the present invention.

Referring to FIG. 9, a specimen measurement cassette 200 may be combined with the specimen-containing unit 100 to constitute the specimen measurement unit 400, as described above with reference to FIGS. 1 to 8, and may include a first accommodation portion S1, a second accommodation portion S2, and a measurement portion S3.

The first accommodation portion S1 may accommodate a first reagent R1, and may provide a space in which a specimen contained in the containment hole 110 of the specimen containment portion 140 reacts with the first reagent R1.

That is, the first accommodation portion S1 may refer to a space in which the specimen reacts with the first reagent R1 with rotation of the specimen containment portion 140 by the rotary force application portion 310 since the specimen containment portion 140 penetrates the first accommodation portion S1.

However, the inflow of the specimen into the first accommodation portion S1 is not limited to being implemented by the specimen containment portion 140. For example, the inflow of the specimen into the first accommodation portion S1 may be implemented using various methods.

The first reagent R1 may be a hemolytic solution for hemolyzing a blood sample as the specimen, and glycated hemoglobin binding material-beads which selectively react with glycated hemoglobin.

For example, the hemolytic solution may be implemented using a buffer solution containing a surfactant, for example, a 20 mM HEPES buffer solution (N-2-hydroxyethylpiperazine-N'-2-ethanesulfonic acid, pH 8.1). Hemoglobin and glycated hemoglobin are present in the hemolyzed blood sample.

The glycated hemoglobin binding material is a material which specifically binds to glycated hemoglobin. For example, the glycated hemoglobin binding material may be at least one selected from the group consisting of boronic acid (BA), concanavalin A (i.e., lectin), and antibodies.

The beads may be at least one selected from the group consisting of a polymeric polysaccharide support such as agarose, cellulose, or sepharose, latex beads such as polystyrene, polymethyl methacrylate, or polyvinyl toluene, and glass beads.

In this case, the particle size of the glycated hemoglobin binding material-beads may be chosen in consideration of a sedimentation time of the glycated hemoglobin binding material-beads bound to glycated hemoglobin after the reaction, and a degree of reaction with glycated hemoglobin.

In summary, the first reagent R1 may include a hemolytic solution for hemolyzing a blood sample as the specimen, and glycated hemoglobin binding material-beads which selectively react with glycated hemoglobin, and the total amount of hemoglobin may be assayed from the blood sample as the specimen reacting with the first reagent R1.

The second accommodation portion S2 may be partitioned from the first accommodation portion S1, and accommodate a second reagent R2.

The second reagent R2 may include a cleaning solution for washing a mixture of the first reagent R1 and the blood sample as the specimen.

Most of hemoglobin (Hb) present in red blood cells of the blood sample as the specimen is typical non-glycated hemoglobin (Ao). In this case, only 4 to 14% of normal hemoglobin reacts with glucose, and is present in the form of glycated hemoglobin (HbA1c).

Therefore, the glycated hemoglobin binding material-beads reacting with the blood sample by means of the first reagent R1 include normal hemoglobin in addition to the glycated hemoglobin. As a result, normal hemoglobin has to be removed to measure an amount of the glycated hemoglobin in blood.

For this purpose, the second reagent R2 may include a cleaning solution for washing normal hemoglobin, which makes it possible to measure an amount of the glycated hemoglobin.

The measurement portion S3 may assay the results obtained by reaction of the blood sample as the specimen with the first reagent R1, and may be arranged under the first accommodation portion S1 and the second accommodation portion S2.

The measurement portion S3 may measure the total amount of hemoglobin in the blood sample when a mixture of the first reagent R1 and the blood sample as the specimen primarily flowing from the first accommodation portion S1 flows into the measurement portion S3. Here, the amount of hemoglobin may be measured by a measurement sensor (not shown) of the specimen measurement body 300, which has the specimen measurement cassette 200 installed therein, using light reflection characteristics.

Here, the measurement portion S3 may include an optical window by which light is reflected through an external optical sensor serving as a measurement sensor, and may have a horizontal cross-section tapering in a downward direction.

Specifically, the measurement portion S3 may have an inclined plane 250 directed downward toward the center thereof, and the inflow of the mixture of the first reagent R1 and the specimen may be implemented by a self-load.

As a result, the inflow of the first reagent R1 into the measurement portion S3 may be implemented by a self-load of the first reagent R1.

Here, the outflow of the mixture of the first reagent R1 and the specimen from the first accommodation portion S1 may be implemented by shifting the first foil tap 220 configured to close a bottom surface of the first accommodation portion S1, and the shifting of the first foil tap 220 may be implemented by the external force application portion 320 of the specimen measurement body 300.

Meanwhile, when the total amount of hemoglobin is measured with respect to the mixture of the first reagent R1 and the specimen flowing in the measurement portion S3, residual solutions other than normal hemoglobin and the binding material-beads bound to glycated hemoglobin may be absorbed by the absorption portion 230 arranged under the measurement portion S3.

By way of example, the absorption portion 230 may be in the form of an absorption pad, but the present invention is not particularly limited thereto.

When the residual solutions other than normal hemoglobin and the binding material-beads bound to glycated hemoglobin are absorbed by the absorption portion 230, the second reagent R2 from the second accommodation portion S2 may flow in the measurement portion S3 by a self-load. Then, an amount of glycated hemoglobin may be measured after the mixture of the blood sample is washed with the second reagent R2 at the measurement portion S3.

Here, the outflow of the second reagent R2 from the second accommodation portion S2 into the measurement portion S3 may be implemented by shifting the second foil tap 240 configured to close a bottom surface of the second accommodation portion S2, and the shifting of the second foil tap 240 may be implemented by the external force application portion 320 of the specimen measurement body 300.

Meanwhile, the amount of the glycated hemoglobin may be measured by a measurement sensor (not shown) of the specimen measurement body 300 using light reflection characteristics, like the measurement of the total amount of hemoglobin from the mixture of blood sample reacting with the first reagent R1.

Subsequently, the amount of hemoglobin may be divided by the amount of glycated hemoglobin to measure a relative amount of glycated hemoglobin in the blood sample as the specimen (Ratio of glycated hemoglobin (%) = glycated hemoglobin/total amount of hemoglobin x 100).

Meanwhile, the above-described spaces for reaction of the specimen with the first reagent R1 and second reagent R2 may be different. Specifically, a space for reaction of the specimen with the first reagent R1 may be the first accommodation portion S1, and a space for reaction of the specimen with the second reagent R2 may be the measurement portion S3.

In addition, the measurement portion S3 may be configured so that the first accommodation portion S1 accommodates the specimen primarily reacting with the first reagent R1, that is, a mixture of the specimen and the first reagent R1, and then accommodates the second reagent R2 after a predetermined time has elapsed.

Meanwhile, the shifting of the first foil tap 220 and second foil tap 240 configured to close bottom surfaces of the first accommodation portion S1 and the second accommodation portion S2, respectively, may be implemented by the external force application portion 320 provided at the specimen measurement body 300.

That is, the first foil tap 220 and the second foil tap 240 may prevent the first reagent R1 and the second reagent R2 from flowing outside. In this case, the first foil tap 220 and the second foil tap 240 may be shifted from the bottom surfaces of the first accommodation portion S1 and the second accommodation portion S2 by the specimen measurement body 300 of the specimen measurement device 10 having the specimen measurement cassette 200 installed therein, so that the first reagent R1 and the second reagent R2 are allowed to flow into the measurement portion S3.

Here, the external force application portion 320 provided at the specimen measurement body 300 may apply an external force to side ends of the first foil tap 220 and the second foil tap 240 in opposite directions. Specifically, the external force application portion 320 may apply a pushing external force to one side end of the first foil tap 220, and may provide a pulling external force to one side end of the second foil tap 240.

However, a plurality of external force application portions 320 may be formed to provide an external force to each of the first foil tap 220 and the second foil tap 240. In this case, the plurality of external force application portions 320 may provide a pulling external force to both side ends of the first foil tap 220 and the second foil tap 240.

Meanwhile, the application of the external force by the external force application portion 320 may be automatically implemented, and also be manually implemented.

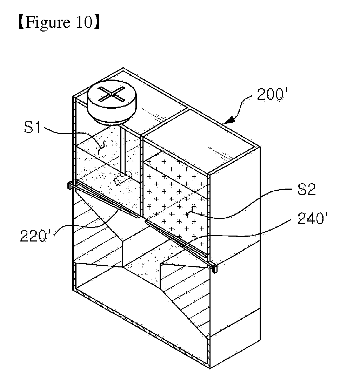

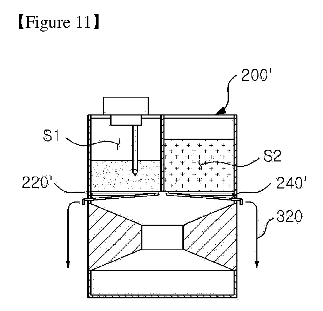

FIG. 10 is a schematic cutaway perspective view showing a modified specimen measurement unit according to one exemplary embodiment of the present invention, and FIG. 11 is a schematic cross-sectional view showing another modified specimen measurement unit according to one exemplary embodiment of the present invention.

Referring to FIGS. 10 and 11, a first foil tap 220' and a second foil tap 240' configured to close bottom surfaces of a first accommodation portion S1 and a second accommodation portion S2 of a specimen measurement cassette 200' may be arranged so that each of the first foil tap 220' and the second foil tap 240' is bent toward the other end.

In this case, the plurality of external force application portions 320 provided at the specimen measurement body 300 may be formed to provide an external force to each of the first foil tap 220' and the second foil tap 240'. For example, the plurality of external force application portions 320 may provide a pushing external force to a lower portion of each of the first foil tap 220' and the second foil tap 240' or provide a pulling external force to an upper portion of each of the first foil tap 220' and the second foil tap 240' so as to shift the first foil tap 220' and the second foil tap 240'.

In summary, the present invention can effectively implement a reaction between the specimen and the reagent in a short period of time with rotation of the specimen-containing unit 100 or 100' by the rotary force application portion 310 provided at the specimen measurement body 300, and can provide convenience to researchers since a place configured to store the first reagent R1 and the second reagent R2 and a place in which the first reagent R1 and second reagent R2 react with a specimen are formed in one structure, that is, a specimen measurement cassette 200.

Also, the mixing between the first reagent R1 and the second reagent R2 can be prevented to accurately assay the results obtained by reaction of the specimen with the first reagent R1 and the second reagent R2.

Further, the shifting of the first foil tap 220 or 220' and the second foil tap 240 or 240' through which the first reagent R1 and the second reagent R2 flow into the measurement portion S3 can be readily implemented by the external force application portion 320, thereby simplifying a configuration of the device itself.

It will be apparent to those skilled in the art that various modifications can be made to the above-described exemplary embodiments of the present invention without departing from the spirit or scope of the invention. Thus, it is intended that the present invention covers all such modifications provided they come within the scope of the appended claims and their equivalents.

## Claims

1. A specimen-containing unit configured to dip a specimen in a reagent for a reaction with the reagent, comprising:
a body portion rotatable by an external force;
an extension portion formed to extend from one surface of the body portion to a predetermined length in a longitudinal direction; and
a specimen containment portion provided at an end portion of the extension portion and having a containment hole formed therethrough while being maintained at a predetermined angle in the longitudinal direction to contain the specimen due to a capillary phenomenon.

2. The specimen-containing unit of claim 1, wherein the extension portion is arranged outside based on the center of one surface of the body portion so that the reagent and the specimen contained in the containment hole are stirred as the specimen containment portion rotates with rotation of the body portion.

3. The specimen-containing unit of claim 1, wherein a lower end portion of the specimen containment portion is sharply formed through a lidding foil portion of an accommodation portion containing the reagent so that the specimen is dipped in the reagent.

4. The specimen-containing unit of claim 1, wherein a direction of the containment hole corresponds to a tangential direction of an imaginary circle formed by an end portion of the extension portion as the extension portion rotates with rotation of the body portion.

5. The specimen-containing unit of claim 1, wherein the specimen-containing unit is formed so that widths of the extension portion and the specimen containment portion correspond to each other.

6. The specimen-containing unit of claim 1, wherein the specimen containment portion is formed integrally with the extension portion.

7. A specimen measurement unit comprising:
the specimen-containing unit defined in any one of claims 1 to 6; and
a specimen measurement cassette comprising a first accommodation portion configured to store a first reagent in which the specimen containment portion is dipped to react with the specimen, a second accommodation portion partitioned from the first accommodation portion and configured to store a second reagent, and a measurement portion configured to assay results obtained by reaction of the specimen with the first reagent.

8. The specimen measurement unit of claim 7, wherein the measurement portion is configured to accommodate the second reagent after a predetermined time has elapsed, after the first accommodation portion accommodates the specimen reacting with the first reagent.

9. The specimen measurement unit of claim 7, wherein the inflow of the first reagent and the second reagent into the measurement portion is implemented by self-loads of the first reagent and the second reagent.

10. The specimen measurement unit of claim 7, wherein the first reagent and the second reagent are prevented from flowing outwards by a first foil tap and a second foil tap configured to close bottom surfaces of the first accommodation portion and the second accommodation portion, respectively, and
the first foil tap and the second foil tap are shifted from the bottom surfaces of the first accommodation portion and the second accommodation portion, respectively, by a specimen measurement device having the specimen measurement cassette installed therein to assay results obtained by reaction of the specimen with the first reagent and the second reagent, so that the first reagent and the second reagent flow into the measurement portion.

11. The specimen measurement unit of claim 10, wherein each of the first foil tap and the second foil tap is arranged so that one end of each of the first foil tap and the second foil tap is bent toward the other end.

12. The specimen measurement unit of claim 7, further comprising an absorption portion disposed under the measurement portion to absorb a mixture of the specimen in which the results obtained by reaction of the specimen with the first reagent are completely assayed.

13. The specimen measurement unit of claim 7, wherein the reaction of the specimen with the second reagent is implemented at the measurement portion.

14. A specimen measurement device comprising:
the specimen measurement unit defined in claim 10; and
a specimen measurement body having the specimen measurement cassette installed therein, configured to assay results obtained by reaction of the specimen with the first reagent and the second reagent, and comprising an external force application portion configured to apply an external force to shift the first foil tap and the second foil tap from bottom surfaces of the first accommodation portion and the second accommodation portion, respectively.

15. The specimen measurement device of claim 14, wherein the external force application portion applies the external force to one side end of the first foil tap and one side end of the second foil tap, respectively, in opposite directions.

16. The specimen measurement device of claim 15, wherein the external force application portion applies a pushing external force to one side end of the first foil tap and applies a pulling external force to one side end of the second foil tap.

17. The specimen measurement device of claim 14, wherein each of the first foil tap and the second foil tap is arranged so that one end of each of the first foil tap and the second foil tap is bent toward the other end, and
the external force application portion applies a pulling or pushing external force to the other side ends of the first foil tap and the second foil tap.

18. The specimen measurement device of claim 14, wherein the specimen measurement body comprises a rotary force application portion configured to apply a rotary force to rotate the body portion after the specimen containment portion is dipped in the first reagent.
